(19) **Europäisches Patentamt / European Patent Office / Office européen des brevets**

(11) **EP 4 311 481 B1**

(12) # EUROPEAN PATENT SPECIFICATION

(45) Date of publication and mention
of the grant of the patent:
**14.01.2026 Bulletin 2026/03**

(21) Application number: **23193484.5**

(22) Date of filing: **03.05.2019**

(51) International Patent Classification (IPC):
**A61B 5/022** (2006.01)     **A61B 5/00** (2006.01)

(52) Cooperative Patent Classification (CPC):
**A61B 5/02241; A61B 5/0053; A61B 5/6843;
A61B 5/6898;** A61B 2560/0462; A61B 2562/0247

(54) **IMPROVED PERSONAL HEALTH DATA COLLECTION**

VERBESSERTE ERFASSUNG PERSÖNLICHER GESUNDHEITSDATEN

COLLECTE DE DONNÉES DE SANTÉ PERSONNELLES AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.05.2018 GB 201807341
15.11.2018 GB 201818626**

(43) Date of publication of application:
**31.01.2024 Bulletin 2024/05**

(62) Document number(s) of the earlier application(s) in
accordance with Art. 76 EPC:
**19727514.2 / 3 787 490**

(73) Proprietor: **LMD IP, LLC
Oklahoma City, OK 73116 (US)**

(72) Inventors:
• **ELLIOTT, Christopher
1015 Lausanne (CH)**
• **JONES, Mark-Eric
1305 Penthalaz (CH)**

• **BAUSER, Philippe
01220 Divonne les Bains (FR)**
• **CLERC, Didier
1870 Monthey (CH)**
• **GAWAD, Shady
1027 Lonay (CH)**

(74) Representative: **Carpmaels & Ransford LLP
One Southampton Row
London WC1B 5HA (GB)**

(56) References cited:
WO-A1-2014/125431      WO-A1-2016/096919
WO-A2-2013/001265      WO-A2-2017/140748
US-A1- 2012 190 944      US-A1- 2014 066 793

• KOOHI IRAJ ET AL: "Coefficient-free blood
pressure estimation based on arterial lumen area
oscillations in oscillometric methods", 2015
IEEE 9TH INTERNATIONAL SYMPOSIUM ON
INTELLIGENT SIGNAL PROCESSING (WISP)
PROCEEDINGS, IEEE, 15 May 2015 (2015-05-15),
pages 1 - 6, XP033168029, DOI: 10.1109/
WISP.2015.7139181

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The invention disclosed herein relates to improvements in the collection personal health data. It further relates to a Personal Health Monitor (PHM), which may be a Personal Hand Held Monitor (PHHM), which incorporates a Signal Acquisition Device (SAD) and a processor with its attendant screen and other peripherals. The SAD is adapted to acquire signals which can be used to derive one or more measurements of parameters related to the health of a user. The computing and other facilities of the PHM with which the SAD is integrated are adapted to control and analyse signals received from the SAD. The personal health data collected by the SAD may include data related to one or more of blood pressure, pulse rate, blood oxygen level ($SpO_2$), body temperature, respiration rate, ECG, cardiac output, heart function timing, arterial stiffness, tissue stiffness, hydration, the concentration of constituents of the blood such as glucose or alcohol and the identity of the user.

A first aspect of the invention relates to adaptation of the PHM and SAD so as to use the fingertip as the body part against which the SAD is pressed or which is pressed against the SAD.

A second aspect of the invention relates to means of collecting and interpreting data from the SAD so as to achieve one or more of: a shorter measurement time; more complete measurement of the pressure through the pulse; and computation of the pressure in arteries other than that at which the measurements are made, such as the aorta.

A third aspect relates to adaptation to a different type of PHM, formed by integrating the SAD with a pair of "Smart Glasses", the latter being a form of a pair of spectacles which incorporates a processor providing, for instance, communications, computing and display capability, together using the cheek as the body part against which the SAD is pressed or which is pressed against the SAD.

A fourth aspect relates to a way of constructing the SAD to reduce cost and improve manufacturability.

A fifth aspect relates to adaptation of the PHM and SAD so allow additional parameters to be extracted from the data, particularly tissue stiffness.

Each aspect of the present invention by itself results in a PHM that is less expensive, easier to use, more accurate and more effective. The aspects of the invention can be used in any and all possible combinations to provide further improvements.

BACKGROUND

WO2013/001265 (PCT1) discloses a PHHM in which a signal acquisition device (SAD) is integrated with a Personal Hand Held Computing Device (PHHCD), such as a cell phone, and is adapted for the measurement of, for example, blood pressure or one or more of several other health-related parameters. The SAD is adapted to be pressed against a body part or to have a body part pressed against it, for example, where the body part is the tip of a finger.

WO2014/125431 (PCT2) discloses several improvements of the aspect described in PCT1, including the use of: a gel to measure pressure; a saddle-shape surface to interact with a body part; corrections for the actual position of an artery relative to the device; and the use of interactive instructions to the user.

WO2014/125355 (PCTG1) discloses improvements to the non-invasive blood analysis disclosed in PCT1 including improvements to the specificity and accuracy of the measurements.

WO2016/096919 (PCT3) discloses several further improvements to the aspects described in PCT1 and PCT2, including improvements to the gel and pressure sensing means, the use of mathematical procedures for extracting blood pressures and other signal processing aspects, a means for identifying the user, improvements to the electrical systems for measurement and several embodiments of test and calibration of the device.

WO2017/140748 (PCT4) discloses further improvements to extracting blood pressure and several other health-related parameters that can be derived from the measured data.

WO2017/198981 (PCTG2) discloses improvements to the aspects disclosed in PCTG1 whereby the device can be built using small and inexpensive components.

PCT1, PCT2, PCTG1, PCT3, PCT4 and PCTG2 are all in the name of Leman Micro Devices SA and are therefore collectively referred to herein as "the Leman applications".

The aspects disclosed herein may also include one or more or any combination of the features disclosed in the Leman applications, including, but not restricted to:

the SAD may comprise a blood flow occlusion means adapted to be pressed against one side only of a body part or to have one side only of a body part pressed against it, a means for measuring the pressure applied by or to the body part, and a means for detecting the flow of blood through the body part in contact with the blood flow occlusion means (PCT1, Claim 1);

where the SAD includes a means for detecting flow of blood, the device may be adapted to detect flow at a range of pressures in any order (PCT1, page 23 lines 4 to 6);

where the SAD includes a means for detecting flow of blood, the means for detecting the flow of blood may employ an oscillometric method (PCT1, Claim 2) or an optical sensor (PCT1, Claim 3);

where a blood flow occlusion means is present, the device may be adapted to give audible or visual instructions to the user to adjust the force with which the blood flow occlusion means is pressed on the body part or with which the body part is pressed onto the blood flow occlusion means (PCT1, Claim 4);

where the device is adapted to provide a blood pressure measurement, it may be adapted to estimate systolic blood pressure (SBP) and diastolic blood pressure (DBP) by fitting the measured data to a theoretical curve that relates blood flow rate to external applied pressure (PCT1, Claim 10);

the device may include a temperature sensor adapted to measure the temperature of a body part (PCT1, Claim 20);

the SAD may be adapted to include a body temperature sensor which is a bolometer and may further include means for displaying the temperature on the screen (PCT2, Claims 51 to 53);

the device may be adapted to provide a measurement of the concentration of an analyte in the user's blood (PCT1, Claim 25);

where the SAD includes a pressure sensor, the pressure may be sensed by means of a flexible and essentially incompressible gel in which is immersed a pressure sensor (PCT2, Claim 1);

the device may be adapted to estimate its height with respect to a fixed point on the subject's body (PCT2, Claims 38 and 39);

the device may be adapted to determine whether the device is in the best position or being used correctly (PCT3, Claim 56);

the device may be adapted to carry out a process to measure a DBP value and a SBP value, wherein the DBP and the SBP values are estimated in such a way that the difference between the measured optical signals and those that would be generated by the estimation of DBP and the SBP values is minimized (PCT3, Claim 8);

the device may include an electrode disposed on or adjacent the SAD, or at least part of the housing is made of an electrically conductive material, wherein the electrode is adapted to transmit electrical signals from the body part of the subject pressed against the SAD (PCT3, Claims 35 to 39);

the device may be adapted to extract one or more features from the signals that is/are correlated with the identity of the subject (PCT3, Claims 80 to 86);

the device may be adapted to carry out test and calibration procedures (PCT3, Claims 96 to 118);

the device may be adapted to find and analyse characteristic features (PCT4, Claim 1 to 22);

the device may be adapted to correct for the position of the body part (PCT4, Claims 23 to 27);

the device may be adapted to detect the mechanical response of the heart to the natural electrical signals which trigger the beating of the heart by holding the device against the chest and processing signals from ECG sensors and an accelerometer (PCT 5, Claims 33 to 37 and 40 to 47);

the device may employ one or more photo-emitters for transmitting light to a body part of a user, wherein the light is green (PCT 2, Claim 62); and

the device may estimate arterial stiffness (PCT4 Claim 41).

## SUMMARY OF THE INVENTION

The various aspects of the present invention are defined in the independent claims set out at the end of this specification.

Preferred features of the various aspects of the invention are defined in the dependent claims set out at the end of this specification. The various aspects of the present invention are described in more detail in the following description. However, the invention in any of its aspects is not limited to any particular feature described hereafter. The scope of the invention is determined by the claims.

## BRIEF DESCRIPTION OF THE DRAWINGS

The present invention is described below, by way of example only, with reference to the accompanying drawings, in which:

Figure 1 shows the typical network of arteries in the fingertip;

Figure 2 shows a typical configuration of the SAD adapted for measurements on the fingertip;

Figure 3a shows a SAD mounted on the back of a Smartphone being squeezed between finger and thumb;

Figure 3b shows a SAD mounted on the side of a Smartphone being squeezed by the tip of the index finger with the hand cupped across the back of the Smartphone;

Figure 4 shows the typical pressure in an artery PINT throughout the cycle of a single heartbeat, referred to above as f(t);

Figure 5 shows the luminal area A as a function of the transmural pressure PTMP, referred to below as g(PTMP);

Figure 6 shows a typical curve of luminal area as a function of the measured pressure, referred to as PM below;

Figure 7 shows the results of a simulation of a deconvolution method, in four parts, wherein;

Figure 7a shows a simulated PINT;

Figure 7b shows a simulated PM;

Figure 7c shows a measured luminal area, where the solid line assumes no noise and the dashed line has noise added to test the algorithm; and

Figure 7d shows the resulting PINT, found by optimising the parameters of the model using measured data;

Figure 8 shows the approximate location of a device when attached to a pair of Smart Glasses;

Figure 9 shows the anatomy of the head, looking at the right ear so the face is to the right of the drawing and the ear lobe is at the bottom left;

Figure 10 is a side elevation of a membrane-covered signal acquisition device (MSAD);

Figure 11 is a plan of the MSAD without the membrane, showing the location of the components;

Figure 12 is a representation of a block of 4 MSADs, ready for test and calibration, before dicing; and

Figure 13 shows the measured ratio of red to green light transmitted as a function of the applied pressure, where the body part is a fingertip.

It should be clearly understood that, for all of the aspects and embodiments, the figures and the descriptions thereof are provided purely by way of illustration and that the scope of the aspects and embodiments is not limited to this description of specific features; rather the scope of the aspects and embodiments is set out in the attached claims.

Aspect 1: Use of fingertip

The Leman applications disclose a SAD that is pressed against a body part or against which a body part is pressed. Many of the details of the specific embodiments disclosed in the Leman applications relate to a SAD which is adapted primarily to interact with the side of an index finger or, for body temperature, the forehead. It has been found that the devices disclosed by the Leman applications are effective but that some people find it difficult to use the side of the finger. The present aspect improves usability and accuracy of the blood pressure measurement.

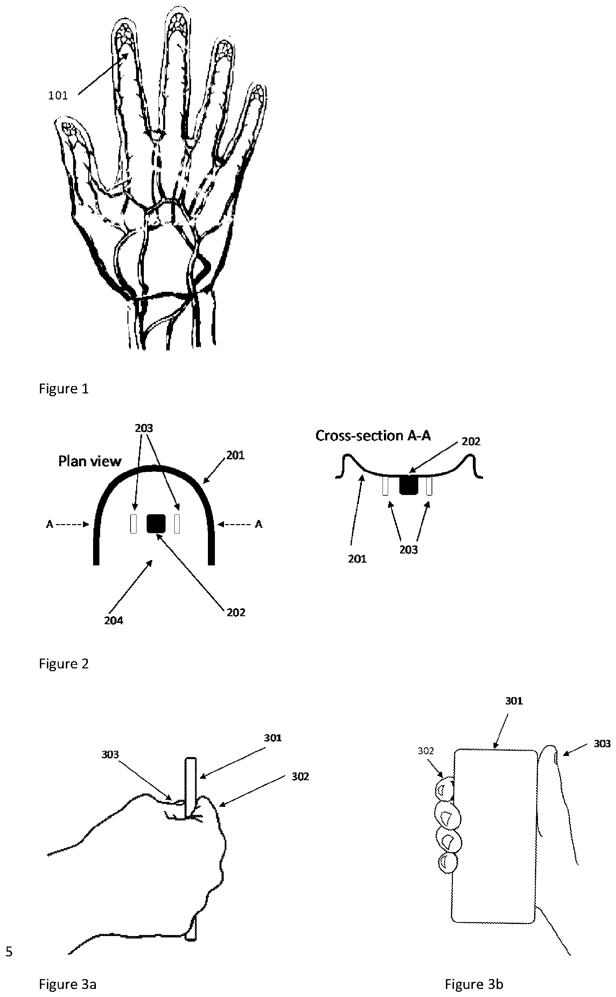

The present invention provides a SAD that is adapted to interact with the fingertip. The SAD may be pressed onto the fingertip or the fingertip may be pressed onto the SAD. Figure 1 shows that the network of arteries is different from the few dominant arteries found, for example, on the side of the finger or at the wrist. Furthermore, the shape of the fingertip is different from other body parts. Preferably, the adaptations include changing the shape of the external surface of the SAD (see paragraph 0026 below); changing the weights used in the analysis (see paragraph 0034 below), changing the estimates of displacement (see paragraph 0035 below) and changing the colour of the light used in the optical sensor (see paragraph 0033 below).

The SAD of this aspect will preferably include at least a pressure sensor for measuring the pressure generated by the interaction of the SAD with the fingertip and a blood flow sensor for measuring the flow of blood through the fingertip so that a measurement of blood pressure can be obtained. However, if the SAD is adapted to measure a different or additional health-related parameter, then different or additional sensors may be included in the SAD, as described in the Leman applications.

Preferably, the SAD of the present aspect may be integrated with a hand held device such as a cell phone.

Figure 1 shows the palmar network of arteries in the hand and the distal palmar arterial arch 101 of the index finger.

In order to be able to take a blood pressure measurement using a device of this aspect of the invention, such as a Smartphone, having an integral SAD, the external surface of the SAD must be adapted to take measurements on the fingertip. Preferably the external surface supports the sides of the fingertip so as to create a more constant pressure field within the fingertip than would be obtained from a flat surface. Preferably, the device is adapted to guide the user to help him or her place the finger correctly over the sensing elements of the SAD.

Figure 2 shows a preferred configuration of the external surface of a SAD of this aspect, in plan view and cross-section. There is a ridge 201 against which the tip of the finger is placed. This ensures that it is correctly located over a pressure sensor 202 and optical windows 203. The cross-section shows how the ridge 201 is formed to support the sides of the finger. The radius of curvature of the fingertip is typically from 6 to 10 mm and the shape of the ridge in this cross-section is chosen so that it provides effective location and squeezing for this range of fingertip radii. The shape of the ridge in the cross-section perpendicular to the cross-section A-A is chosen so that smaller fingers are naturally located lower down the ridge, so ensuring that the centre of the fingertip remains over the pressure sensor 202 and optical windows 203.

Preferably, there is provided a flat area 204 within and preferably beyond the ridge to support the fingertip.

Preferably, the optical windows 203 lie flush with the surface rather than sloping as in the saddle shape surfaces described in the Leman applications.

Preferably, as shown in Figure 3a, the SAD is located on the back of a Smartphone and is operated by pinching the Smartphone between the right index finger 302 and the thumb 303. The thumb 303 is therefore placed on the screen of the Smartphone 301. Most Smartphones have a screen that is at least touch-sensitive and is therefore able to locate where a finger is touching the screen. Some can also measure the force that is applied by the finger that is touching the screen. Preferably, the processor of the Smartphone (or any other device with which the SAD may be integrated) is adapted to use the touch-sensitive screen to indicate where the user should place his or her thumb to ensure the correct position of the index finger and to check that the thumb is so located.

The force generated by the thumb may be used as a pressure sensor, either because the screen is force-sensitive or because the screen detects the area of contact with the thumb, which spreads as the thumb presses harder. The force estimate is indicative of the pressure being applied to the SAD of known area and therefore provides an independent estimate of that pressure. Said estimate may be used to complement, check or replace the measurement made by the pressure sensor in the SAD. It may also be used to ensure that the user is applying approximately the correct pressure when making measurements that are affected by that pressure, such as blood oxygen concentration, using a device that does not include a pressure sensor.

In an alternative embodiment, as shown in Figure 3b, the SAD is mounted on the left side of the screen of the Smartphone and the user holds the Smartphone with the right hand cupped across its back, pressing the tip of the index finger on the SAD. In this embodiment, the external surface shown in Figure 2 is omitted.

As shown in Figure 1, the fingertip has a network of arteries rather than a single dominant artery as is encountered with other body parts. Some of those may be to the side of the pressure sensor and, if the user pushes the finger with a sideways or shearing motion, may cause the pressure in these regions to be different from that over the pressure sensor. Preferably, the SAD is adapted to use at least one LED that emits light of a colour that is strongly absorbed in the tissue of the finger, such as green (PCT 2, Claim 62) so that the absorption detected by the SAD is primarily from the region close to the optical sensor and therefore over the pressure sensor. Preferably, the differences between the signals from this LED and those from the other LED, which is less absorbed by the tissue, are used to correct for any residual effects of non-uniform pressure (PCT4, Claims 23 to 27).

It is preferable that the processor is adapted to accommodate the differences in the signals obtained from the fingertip from those obtained from other body parts. For example, the amplitude of the pressure pulses is smaller. It is preferable that the weights used to find the weighted mean of separate estimates of blood pressure (PCT4, Claim 19 et seq) are adapted to suit the accuracy of each of the separate estimates when used on the fingertip. PCT4, Claim 23 et seq disclose adaptations to allow the measured values to be corrected for the position of the body part.

Preferably, these are adapted so that estimates are made of the displacement of the sensor with respect to the position of the artery, the rotation of the fingertip and/or the size of the fingertip.

Aspect 2: Means for collecting and interpreting data

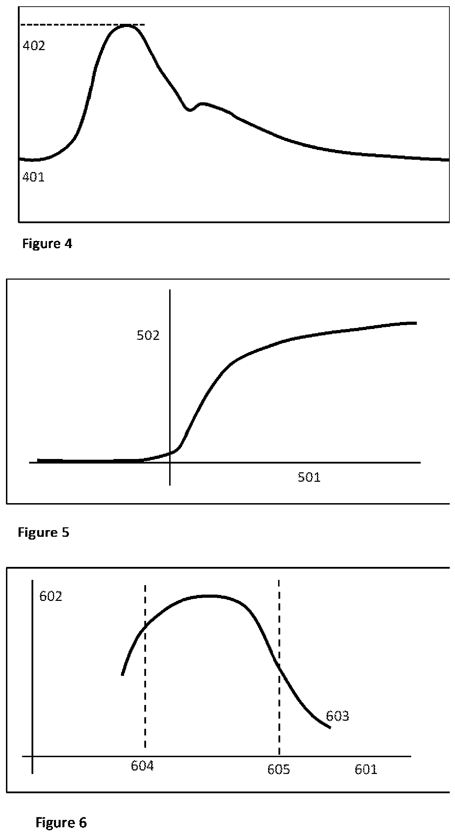

The instantaneous pressure of the blood in an artery varies during each pulse cycle and approximately repeats on successive cycles. The typical pressure as a function of time is plotted in Figure 4, where the vertical axis is the arterial blood pressure and the horizontal axis is time. The pressure goes from diastolic 401 to systolic 402 and then falls, with a perturbation 403 known as the dicrotic notch.

The difference between the pressure of the blood inside the artery (PINT) and the pressure in the tissue outside the artery POUT causes the arterial wall to stretch until the pressure change across the artery wall (Trans-Mural Pressure PTMP) is equal to PINT - POUT. The material of the artery wall is elastic but non-linear in that its stiffness depends on how much is has stretched.

Figure 5 illustrates this by plotting the luminal area A on the vertical axis 502 against PTMP on the horizontal axis 501. When PTMP is negative, the pressure outside the artery is greater than the pressure inside and the artery collapses ("is occluded" in medical terms) to close to zero luminal area. When the pressure inside exceeds the pressure outside, the artery opens ("is patent" in medical terms). As PTMP increases, the artery becomes stiffer and so the luminal area increases less with increase of PTMP. Various equations have been proposed to describe this behaviour of the arterial wall, including by Langewouters (Clin Phys Physiol Meas 1986, Vol. 7, 1, 43-55), Drzewiecki (Annals of Biomed Eng, Vol. 22, pp. 88-96, 1994) and Bank (Circ Res. 1995 Nov;77(5):1008-16). The Leman applications approximate these laws by a power law of the form $A = PTMP^k$ where k is of the order of 0.3 to 0.6.

The instantaneous luminal area determines part of the absorption of light passing through the tissue surrounding the artery. This is the principle of the pulse oximeter and is used in the Leman applications as the optical signal to determine the change in area when the artery goes from occluded to patent. The external area of the artery also increases when the artery goes from occluded to patent, although not by as much as the luminal area because the wall thins as it stretches. This gives rise to a pressure pulse in the tissue that is used by conventional oscillometric sphygmomanometers, and also in the Leman applications, to determine the change in area.

Most automatic sphygmomanometers determine the systolic and diastolic arterial blood pressures from a curve of the change in area on each pulse cycle as a function of POUT. Figure 6 shows such a curve where the horizontal axis 601 is POUT, the vertical axis 602 is a measure of the change of luminal area, the measured points from many pulse cycles lie on the line 603 and the diastolic pressure 604 and systolic pressure 605 are shown by dotted lines that pass through points on the curve 603 determined by empirical rules.

The devices disclosed in the Leman applications also use this approach as well as a complementary approach based on the time through the pulse cycle when the artery becomes patent and when it is occluded. In the Leman applications, the user presses the device against a body part or presses the body part against the device. The pressure sensor in the device measures the pressure in the body part by transmission of that pressure through the skin. The optical components in the device detect the absorption of light by the blood in arteries and hence derive an estimate of the luminal area.

All of these methods have two limitations:

they only find the two values of the arterial pressure, systolic and diastolic; and

they use only a small fraction of the data that may collected throughout the pulse cycle.

The present aspect addresses both of these limitations. This is valuable for two reasons: there is considerable medical value in knowing the pressures at points in the arterial system other than the one at which the measurements are made, in particular in the aorta. For example, Stergiopulos published "Physical basis of pressure transfer from periphery to aorta" (Am J Physiol Heart Circ Physiol 274:H1386-H1392, 1998) showing how the full waveform of the peripheral arterial pressure pulse may be transformed to find the aortic pressure. This is not possible if the waveform is only known at systolic and diastolic pressure; and use of more of the data allows less of it to be collected, so reducing the measuring time, and allows noise and systematic perturbations to be suppressed, so improving accuracy.

The SADs disclosed by the Leman applications are effective and accurate and meet the objective that they should be suitable to be integrated into a cell phone. They measure the systolic and diastolic pressures by analysing data that are collected at a range of applied pressures.

The present aspect is referred to as Model-Based Optimisation (MBO). It applies the mathematical process of optimisation to extract an accurate estimate of the parameters of a model of the waveform of the arterial pressure pulse from the values of A that are inferred from the optical signal.

In order to illustrate the application of optimisation, an example is described. The optimisation process described here is one of many ways of solving for the pressure wave. Others are known to a person skilled in the art.

Assume that:

the instantaneous pressure in the artery PINT = f(t) where t is the time through the pulse cycle;

the instantaneous luminal area of the artery A = g(Pint - Pout);

the instantaneous value of POUT is the measured pressure applied to the tissue PM; and

the instantaneous optical signal measured by detecting the light that has passed through the tissue surrounding the artery S(t) = u A(t) + v where v is a quasi-static contribution due to the light that passed without being absorbed by the blood in the artery.

It can then be written that:

$$S = u\,g(f(t)-P_M) + v \qquad\qquad \text{Equation 1}$$

If sufficient measurements of S and PM are made, optimisation may be used to find f(t) and thus PINT throughout the beat of the heart.

In order to illustrate the aspect, f(t) may be represented by a simple model that uses six parameters:

systolic and diastolic pressure;

rate of rise before and fall after systole;

time and amplitude of the dicrotic notch;

The MBO only has to find these six values.

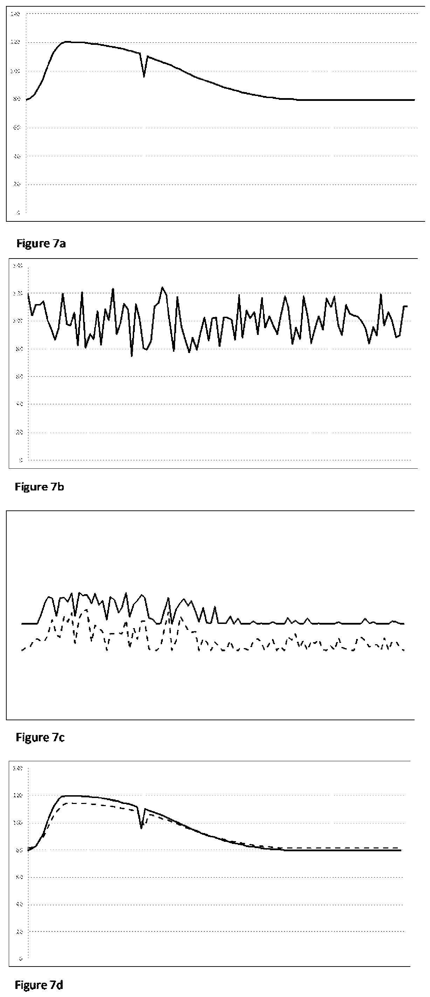

This exemplary optimisation process can be illustrated using simulated data, assuming that the data were obtained by the user pressing a fingertip against the sensor of the type disclosed in the Leman applications. Typical values for systolic pressure (120 mmHg) and diastolic pressure (80 mmHg) were assumed, together with typical values for the other parameters that make up f(t). The resulting model pressure through the pulse cycle is shown in Figure 7a. It is apparent that even this simple six-parameter model can create a waveform with much of the complexity of Figure 4, and one or two more parameters or a more realistic equation defined by those parameters would allow an even more precise alignment.

The user of the system is instructed to apply a pressure of 100 mmHg but in practice muscle action causes a random pressure, centred on 100 mm Hg, to be applied as shown in Figure 7b.

A power law has been assumed for the relationship between area and PTMP, as in the Leman applications, with an extension as in Figure 5 so that the area does not reach zero until PTMP of approximately -20 mmHg.

The S(t) that would be generated by that set of assumptions if there were no noise in the measurement system is shown by the solid line in Figure 7c. Random noise is then added so the measured value of S(t) is as shown by the dotted line in Figure 7c (displaced to be easier to see) on the graph).

The optimisation process estimates a set of parameters for f'(t) and hence finds a simulated resulting S'(t). It then refines the estimated parameters to find the simulated set that minimises the error, where:

$$\text{Error} = \Sigma[S(t) - S'(t)]^2 \qquad\qquad \text{Equation 2.}$$

This is the sum of the squared differences.

Figure 7d shows the resulting pressure wave and the true wave for comparison. Even though there is considerable noise in the data and in the pressure applied by the user, the reconstruction is very accurate. Preferably, the accuracy is improved by standard optimisation techniques that are well-known to a person skilled in the art, such as:

weighting the error function of equation 2 to make most use of the significant data points;

optimising equation 2 to use the absolute value of error and the power thereof;

optimising the simple parametric model shown in Figure 7a by including additional parameters or different dependency on those parameters, to give a more realistic function, such as that shown in Figure 4; and

optimising the noise model, for example to avoid the occurrence of negative luminal area in the model.

The simulation is deliberately simplified to illustrate the aspect. It does not include finding the quasi-static contribution to S(t) referred to as v in Equation 1 and it has only taken data from one pulse cycle. Preferably, this aspect uses data from several pulse cycles and preferably instructs the user to change the target pressure to ensure that a range of values of PM is created, in any order.

Preferably, the optimisation uses the techniques of machine learning, as used in artificial intelligence. It finds the parameters of a model that represents the instantaneous pressure throughout the pulse.

If the pressure field varies across the field of view of the optical system, the measured data is "blurred" because it is a sum of the behaviour of the artery at a range of POUT. This can reduce the effectiveness of the optimisation or require more parameters to be taken into account (PCT4 claims 8 to 12). Preferably, the data used in optimisation are obtained using an optical sensor that uses at least one LED with a wavelength that is strongly absorbed in the tissue, such as a green LED (PCT 2, Claim 62), to reduce the range of pressures that are encountered.

The estimation of SBP and DBP by any of the other approaches described in the Leman applications may be carried out using the same data as is used for the optimisation. These can provide one or more independent estimates of SBP and DBP that can be used either to increase the accuracy of the optimisation or to reduce the amount of data, and hence measurement time, that is needed.

Aspect 3: Use of cheek

The Leman applications are agnostic as to the body part which is pressed against the sensor or against which the sensor is pressed. The devices are in some cases adapted to specific body parts, such as the side of the finger. The present aspect provides a SAD that is adapted to interact with the cheek in front of the ear. The SAD will be pressed on the external carotid artery.

The SAD will preferably include at least a pressure sensor for measuring the pressure generated by the interaction of the SAD with the cheek and a blood flow sensor for measuring the flow of blood through the cheek so that a measurement of blood pressure can be obtained. However, if the SAD is adapted to measure a different or additional health-related parameter, then different or additional sensors may be included in the SAD, as described in the Leman applications. The personal health data collected by the SAD may include data related to one or more of blood pressure, pulse rate, blood oxygen level ($SpO_2$), body temperature, respiration rate, ECG, cardiac output, heart function timing, arterial stiffness, tissue stiffness, hydration, the concentration of constituents of the blood, such as glucose or alcohol, and the identity of the user.

The SAD of the present aspect may be integrated with a pair of "Smart Glasses", the latter being a form of a pair of spectacles which incorporates a processor providing, for instance, communications, computing and display capability.

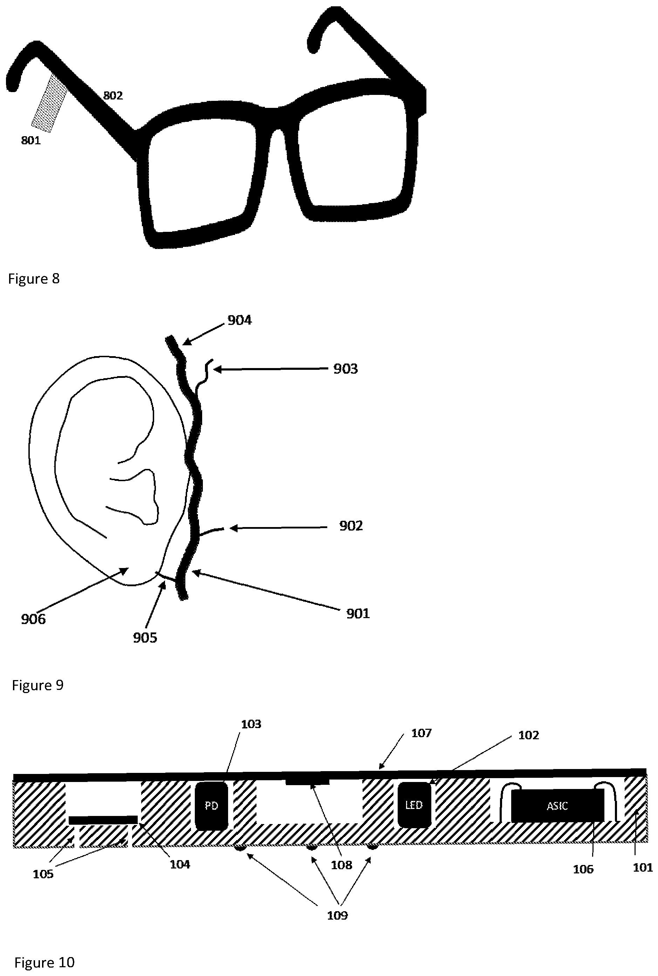

Figure 8 shows the approximate location of a SAD 801, close to the ear on one of the arms 802 of a pair of Smart Glasses which together constitute a PHM. The SAD 801 hangs below the arm, close to the external carotid artery which runs just under the skin, approximately vertically and close to the ear on the side towards the face.

Figure 9 shows the anatomy, looking at the right ear so the face is to the right of the drawing and the ear lobe 906 is at the bottom left. The external carotid artery 901 runs up from the neck. It has branches: the posterior auricular artery 905, the maxillary artery 902 and the transverse facial artery 903, and then becomes the superficial temporal artery 904.

The SAD 801 has an external surface which is flat. The surface of the gel or other pressure sensing means remains co-planar with the remainder of that flat surface. The user operates the Smart Glasses including the SAD 801 by pressing the SAD 801 with a finger against the cheek and varies the force applied in accordance with audible or visual instructions that are generated by the Smart Glasses in order to occlude the external temporal artery. It is preferable that the weights used to find the weighted mean of separate estimates of blood pressure (PCT4, Claim 19 et seq) are adapted to suit the accuracy of each of the separate estimates when used on the cheek. PCT4, Claim 23 et seq. discloses that the measured values may be corrected for the position of the body part. Preferably, estimates are made of the displacement of the sensor with respect to the position of the artery and the extent to which the flat external surface of the SAD is not co-planar with the cheek.

A means for detecting the electrical signal which initiates systole (PCT1, Claim 15, PCT3 Claims 35-39) preferably detects the electrical signal between the cheek and the finger that is being used to press the SAD 301 against the cheek. Alternatively, it may detect the signal between the fingers of the two hands if a second electrode is located on the arm of the Smart Glasses that does not carry the SAD and the finger of the other hand is pressed against this.

A temperature sensor, as described in PCT1, may also be included. Preferably, the temperature sensor measures the temperature of the surface of the skin touching the SAD rather than the emitted infra-red radiation as disclosed in PCT2, Claims 51 et seq. This is because the skin temperature over the external carotid artery is similar to that over the temporal artery and therefore there is no need for a separate temperature sensing procedure. The parameters for compensating for ambient temperature (PCT1, Claim 23) may be optimised for the different location.

The blood analyte sensor disclosed in PCT1, Claims 25 to 29, PCTG1 and PCTG2 can also be used in a SAD adapted to be pressed against the cheek. One possible embodiment of the aspect includes two SADs, one on each arm of the Smart Glasses, one of the SADs being adapted to measure blood pressure, temperature and related parameters and the other being adapted to measure one or more blood analytes. If this is done, it is preferable that an electrical signal is measured between the fingers of the two hands, one of which is pressing on each device.

Aspect 4: Construction of the SAD

The SADs disclosed by the Leman applications are effective and accurate and meet the objective that they should be suitable to integrate into a cell phone. However, cell phones are manufactured in quantities of hundreds of millions and the price of their components is critical. The SADs disclosed in the Leman applications are complicated to manufacture, which increases their cost. The present aspect reduces their cost.

The present aspect relates to a membrane-covered signal acquisition device (MSAD) for collecting personal health data. It further relates to a Personal Health Monitor (PHM), which may be a Personal Hand Held Monitor (PHHM), including the MSAD. The MSAD is adapted to acquire signals which can be used to derive one or more measurements of parameters related to the health of a user. Preferably, the MSAD is integrated with a cell phone (also known as a mobile phone), such as a Smartphone. The computing and other facilities of the PHM with which the MSAD can be integrated are adapted to control and analyse signals received from the MSAD. The personal health data collected by the MSAD may include data related to one or more of blood pressure, pulse rate, blood oxygen level ($SpO_2$), body temperature, respiration rate, ECG, cardiac output, heart function timing, arterial stiffness, tissue stiffness, hydration, the concentration of constituents of the blood, such as glucose or alcohol, and the identity of the user.

According to the present aspect, there is provided a membrane-covered signal acquisition device (MSAD) comprising:

a substrate, such as a printed circuit board (PCB) or an integrated circuit, which includes electronic components required for the operation of the MSAD and which has an upper and a lower surface;

at least one well in the upper surface of the substrate;

a sensor located in the well; and

a flexible membrane covering the well.

Figure 10 shows a side elevation of an MSAD of the present aspect in which there is a multilayer PCB 101 which has five wells in it. In each of the wells is, respectively, one or more light emitting diodes 102 (LEDs), one or more photodiodes 103, a micro-electro-mechanical system (MEMS) thermopile 104 and an application-specific integrated circuit (ASIC) 106. The well containing the thermopile has in its lower surface two vents 105. The upper surface of the PCB, including all the wells, is covered by a membrane 107 of a stiff, deformable material to the underside of which is bonded a piezo-resistive strain gauge 108. There may be a vent similar to vents 105 to the well under said strain gauge. Electrical connections 109 are made on the underside of the PCB.

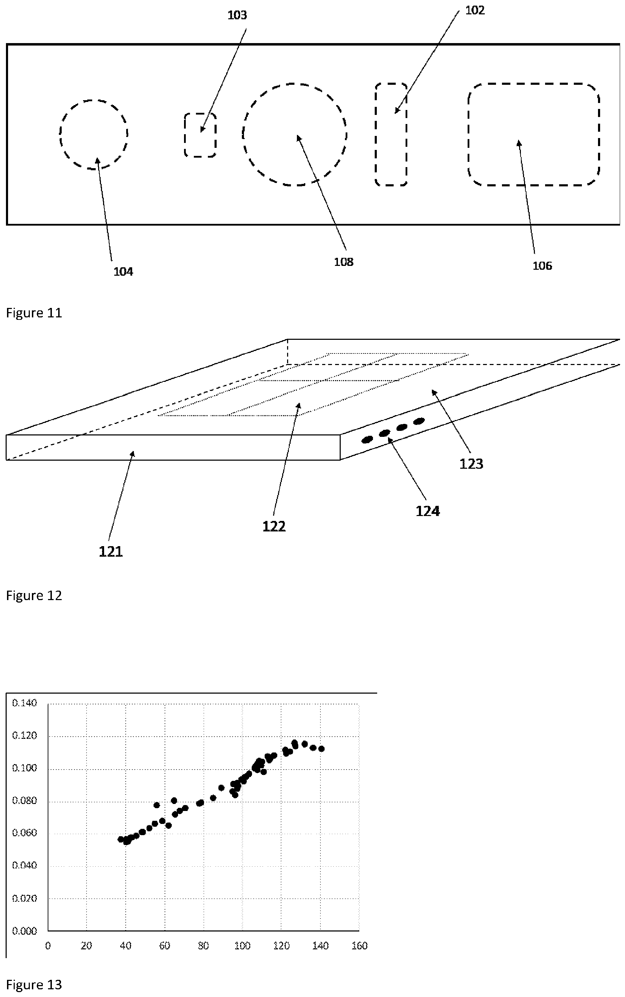

Figure 11 shows a plan view of the MSAD which shows the location of all of the components. The membrane is absent from this drawing.

Preferably the MSAD is assembled by:

soldering or wire bonding all of the components into the appropriate wells in the upper surface of the PCB;

gluing the membrane to the upper surface of the MSAD, using conducting glue where appropriate to make electrical connections to the strain gauge(s); and

flushing the opening surrounding the MEMS thermopile to remove any fumes from the glue and replace them with a suitable inert gas such as dry nitrogen or argon, then sealing the vents.

Alternatively, the PCB may be split so that there is a thin PCB on which the components are mounted and a second PCB glued over it to form the wells. This allows the components to be connected without having to operate inside the wells.

The substrate may merely provide support for components of the MSAD.

Preferably, the substrate provides electrical connections for electrically connecting the sensor and the electronic components.

For instance, the substrate may be a PCB which has printed on it electrical connections to which the sensor and the electronic components are electrically connected. The PCB may also have electronic components embedded in it. Alternatively or additionally, electronic components may be present in a further well or wells in the upper surface of the substrate and be connected together by the electrical connections of the PCB.

Alternatively, the substrate may comprise an integrated circuit where some or all of the electrical connections and electronic components are built into the integrated circuit.

The MSAD includes electrical connections for connecting the MSAD to a device which, together with the MSAD, forms a PHM. The MSAD is preferably configured for physical and electrical connection to a cell phone so that the signals produced by the sensor can be processed by the processor of the cell phone.

The substrate may include more than one well in its upper surface, in which case the flexible membrane covers all wells present in the substrate.

If there is more than one well, in at least one of the wells there will be a sensor.

Each well in which there is a sensor includes electrical connections for controlling the sensor and for transmitting signals from the sensor to a processor. In each such well, there may also be one or more electronic components required for the operation of the sensor.

Any well in which there is no sensor may have in it one or more electronic components required for the operation of the MSAD.

The size and shape of each well is determined by the sensor and/or component(s) which are in it.

The sensor(s) or electronic component(s) present in the or each well do not need to be in contact with the well. They only need to be electrically connected to the other components of the MSAD and connected or connectible to a processor. For instance, a sensor may be attached to the face of the membrane facing towards the lower surface of the substrate.

First Embodiment

In a first embodiment of the present aspect, the MSAD is adapted to measure the blood pressure of a subject. In this case, in one well, one or more strain gauges is/are mounted on the surface of the membrane facing the lower surface of the substrate and is/are electrically connected to the electronic component(s) of the MSAD, for instance by means of electrically-conductive threads printed on the surface of the membrane and electrically-conductive adhesive between the membrane and the substrate. In this embodiment of the aspect, the MSAD also includes a blood flow sensor.

In use, a body part is pressed against the membrane over the strain gauge(s) or the membrane over the strain gauges is pressed against a body part. The pressure between the body part and the membrane causes the strain gauge(s) to bend. The deformation of the strain gauge(s) creates an electrical signal related to the pressure between the body part and the membrane. This allows the measurement of the pressure within the skin of the body part. At the same time, the blood flow sensor produces an electrical signal related to the flow of blood in the body part. These electrical signals are processible by the processor of a PHM to produce a measurement of blood pressure. Processing of such pressure and blood flow signals is described in detail in the Leman applications and a PHM of the present aspect may use the processing methods described therein or any other suitable processing method to derive a measure of blood pressure.

Preferably, the membrane is glued or clamped across the well and has mounted on it one or more resistive strain gauges. Preferably, the or each strain gauge is screen printed onto the membrane using a piezo-resistive material, such as ruthenium dioxide embedded in an epoxy matrix, typically 10 to 20 microns thick. Preferably, the or each strain gauge has silver connecting pads with silver/palladium interconnections to the substrate. This is a mature technology known to a person skilled in the art.

Preferably, the upper surface of the membrane is essentially flat, both within the pressuresensitive area and substantially beyond it, to achieve an accurate measurement of pressure in the body part. In some embodiments of the aspect, outside the area which is adapted to contact the body part, the surface of the membrane then curves to match the shape of the body part to create a more even pressure field.

The well may be vented to prevent the deformation changing with atmospheric pressure. Preferably, the membrane is made of a material with a Young's modulus of from 0.1 to 1.0 GPa and has a thickness of from 100 to 500 micron. It is mounted over the well, which is preferably circular, and its edges firmly attached to the substrate. For a circular hole, the deformation Y of the middle of the membrane over the well is given by:

$$Y = 0.171 \, p \, r^4/(t^3 \, E) \qquad\qquad \text{Equation 3}$$

where p is the pressure exerted on the membrane, r is the radius of the hole, E is the Young's modulus and t is the thickness of the membrane. The strain S has a maximum at the middle of the membrane which is given by:

$$S = 3 \, p \, r^2/(4 \, t^2 \, E) \qquad\qquad \text{Equation 4}$$

Preferably, the displacement is not greater than 20 micron. The strain is up to 0.5% and so is easily measured with conventional strain gauges.

Preferably, the blood flow sensor comprises a light source, such as an LED, located in a well of the substrate and adapted to transmit light to an area above the strain gauge(s) where, in use, the body part will be located, and a photodetector, also located in a well of the substrate and adapted to receive light reflected or refracted by the body part. The light source and the photodetector may be in the same well but are preferably in different wells and may or may not be in the well in which the strain gauge(s) is/are located. There may be multiple light sources and/or multiple photodetectors.

Where the blood flow sensor uses light transmission, the membrane is transparent to light at the relevant wavelength(s). Suitable wavelengths of light and arrangements for the light sources and photodetectors are described in detail in the Leman applications and a MSAD of the present aspect may use any of the wavelengths and arrangements described therein or any others that are suitable.

Second Embodiment

According to a second embodiment of the present aspect, there is provide a MSAD as described in the first embodiment of the aspect but which is not intended to be used to measure blood pressure and so the strain gauges and associated electronics are not necessarily present. Such an MSAD may be adapted to measure blood flow, from which such health related data as pulse and arrhythmia can be derived. Alternatively or additionally, the MSAD of this embodiment of the aspect may be adapted to provide measurements of the concentrations of analytes, such as oxygen, to provide a measure of blood oxygen level ($SpO_2$), water, to provide an indication of hydration, glucose, for use by, for instance, diabetic subjects, and alcohol.

Some of the parameters related to health that are measured by the SADs as disclosed by the Leman applications and the MSAD disclosed in this application are measured by finding the differential absorption of light in a body part:

at more than one wavelength; and/or

at the time of diastole when the artery is small and at the time of systole when it is large.

For example, the measurement of $SpO_2$ relies on two optical wavelengths to distinguish oxygenated and unoxygenated blood and the Leman applications disclose other optical signals that can be used to measure total haemoglobin, glucose, alcohol or another analyte in the blood. The measured absorption is affected by the pressure that is applied to or by the body part and by the way in which that the pressure varies within the field of view of the optical signals. It is necessary to maintain a reasonably controlled pressure in order to obtain accurate measurements.

The SADs disclosed in the Leman applications and the MSAD according to this embodiment of the aspect preferably include a pressure sensor in order to increase the accuracy of the optical measurements even if no measurement of blood pressure is required. This permits the optical measurement to be made at a controlled pressure.

The pressure sensor may be as disclosed as the first embodiment or may be one of the various pressure sensors disclosed in the Leman applications.

Third Embodiment

According to a third embodiment of the present aspect, one of the sensors in the MSAD is a temperature sensor, such as a MEMS thermopile. Preferably, the temperature sensor is sensitive to infrared light, in which case the membrane is transparent to infrared light.

The accuracy of some types of temperature sensors, such as thermopiles, can be affected by the gas that surrounds them. The process of gluing the membrane on to the substrate might release gas into the well containing the temperature sensor. Preferably, there is provided one or more vents that may be used to flush out the gas after applying the membrane and through which a preferred gas may be introduced, after which the vents may be sealed.

Fourth Embodiment

According to a fourth embodiment, the present aspect provides a membrane-covered signal acquisition device (MSAD) comprising a substrate, such as a printed circuit board (PCB) or an integrated circuit, which includes electronic components required for the operation of the MSAD and which has an upper and a lower surface and a membrane covering the upper surface of the substrate, wherein;

the upper surface of the membrane, remote from the substrate, is electrically conductive and electrically connected to the electronic components of the substrate but otherwise electrically isolated; and

the substrate includes an exposed electrical contact electrically connected to the electronic components of the substrate but otherwise electrically isolated

so that, in use, a subject may place one part of the subject's body in contact with the membrane and another body part in contact with the contact and the MSAD is adapted to derive signals related to the electrical activity of the subject's body.

In use, a PHM including a MSAD according to the fourth embodiment of the aspect is adapted to measure such parameters as respiration rate, ECG, cardiac output and heart function timing.

Fifth Embodiment -Testing and Calibration

Preferably, a MSAD according to any one of the four embodiments of the aspect is manufactured by producing a block of MSADs and then the block is cut to form individual MSADs. Preferably, each of the MSADs in the block is tested and calibrated while in the block. By this means, the process of testing and calibration is simplified and made less expensive. Preferably, the block consists of 100 to 200 MSADs, the exact number depending on the precision with which the membrane is formed and attached.

Figure 12 is a representation showing four MSADs as a single block 121. The four MSADs are marked by the dotted lines 122. There is a surround 123 to the block which contains no MSAD. Electrical connectors are provided on the side or under surface of the block.

It is desirable that it should be possible to manufacture, test and calibrate the SADs disclosed by the Leman applications at low cost. The MSADs of the present application can be manufactured at reduced materials and assembly costs, but this is of little value if test and calibration is expensive, taking into account the desirability of being able to produce MSADs at a rate of 3 or more every second. Preferably, test and calibration is carried out on many devices simultaneously.

Calibration requires:

the response of the MSAD to be measured as a function of the pressure applied to any pressure sensor and its temperature;

the wavelengths of any light source(s) to be measured; and

the response of any temperature sensor to radiant temperature to be measured.

In order to do this, many MSADs may be made as a single block and only diced into individual sensors after test and calibration. Preferably, there is a surround to the block which contains no MSAD. Connectors are provided on the side or under the surface of the block for connection to a calibrating system.

Preferably, the membranes in the individual MSADs are derived from a single sheet across the top surface of the block, with any strain gauges printed on to it at the appropriate locations.

Preferably, the block has pegs or similar keys to ensure the correct location of the sheet over the block.

The connections to each MSAD are accessible while it is part of the block so each may be tested and calibrated individually.

Alternatively, the MSADs may be connected together to allow a simpler interface to the calibration system. If present, any ASIC in each MSAD has a bus connection, preferably I2C. It is preferable that all MSADs have the same bus address so that each individual MSAD may be installed in a mobile phone or other PHHCD using the same software. For test and calibration, it is necessary to address each of the MSADs in a block individually and to read the data that each ASIC generates. Preferably, the ASIC in each MSAD has a plurality of address inputs configured so that they may be pulled high or low by an external input but default to a normal state in the absence of such an input.

Each MSAD in the block is configured to pull the inputs of the MSAD that lie downstream of it to a unique address. This ensures that each MSAD is uniquely addressable while in the block but, after the block is diced, all of the MSADs revert to the same default address. The outputs of the ASICs are strapped together in the block so that a single input may read any of them.

Preferably, each MSAD has a unique identifier that may be read via the bus. Preferably, the pressure calibration is effected by clamping a pressure vessel on to the surround and then applying controlled pressures to the pressure vessel and hence the pressure sensors. After test and calibration, the blocks are diced to yield individual MSADs.

Sixth Embodiment - Self-Test

In any MSAD of the present aspect or any SAD of any one of the Leman applications which includes a temperature sensor, errors can arise if the membrane of the MSAD or a window covering the temperature sensor of the SAD is damaged or becomes dirty. The present aspect therefore further provides a method for self-testing a MSAD or SAD that uses a thermopile bolometer as its temperature sensor so that such errors can be detected and avoided.

Any dirt on or damage to the membrane or window over the temperature sensor, if present, will reduce the sensitivity of the temperature sensor to thermal radiation and cause an error to its temperature measurement. The sensitivity may be checked at appropriate intervals by directing the temperature sensor at a surface and causing the cold junction to be heated using the heat dissipated by any light source present. There should be no change in the apparent temperature of the surface because the temperature of the cold junction of the thermopile is measured and used to compensate the measured temperature. Although this approach might not be sufficiently accurate for an absolute calibration of the temperature sensor, changes in the calibration measured by this technique can be detected and used to warn the user to clean the surface of the membrane or window.

The present aspect further provides a PHM, preferably a PHHM, more preferably a cell phone including an MSAD of the present aspect, in all its embodiments, integrated with or connected to the PHM.

All embodiments

In all embodiments of the present aspect, the membrane creates a water-tight and dusttight seal and can act as a window for components of the MSAD.

Preferably, the material used for the membrane extends to cover substantially the whole upper surface of the substrate. Advantageously, the material is transparent at the wavelengths used for any optical sensors (infra-red from around 5 micron to 12 micron for the temperature sensor, visible through to near infra-red for the optical sensor), robust enough to withstand use, chemically inert and, particularly, non-cytotoxic.

Preferably, the membrane is made of high density polyethylene, more preferably of ultra high molecular weight high density polyethylene.

The outer surface of the membrane may be treated to absorb UV light, which is known to cause deterioration of polyethylene and other materials.

It will be appreciated that an MSAD of the present aspect may include the features of any or all of the embodiments of this aspect.

Aspect 5: Further parameters that may be extracted from the data

The stiffness of the tissue is an indicator of hydration and may indicate health more generally. The data derived from the SAD and PHM disclosed in the Leman applications and the aspects of this application provides several independent indications of tissue stiffness.

Independent indications obtained by each of the embodiments disclosed here may be combined and, if necessary, calibrated by correlation with independent measurements of the properties, including hydration, so as to extract an estimate of the stiffness and/or hydration of the tissue.

First embodiment

PCT4, Claim 25 et seq disclose that measured values may be corrected for the position of the body part. There are several independent estimates, making use of data derived from optical sensors and from pressure sensor(s) and using data derived at a range of applied pressures. They each depend on:

the position of the body part with respect to the sensors; and

the mechanical properties of the tissue of the body part and, in particular, the propagation of pressure pulses by the tissue.

In a first embodiment, empirical combinations of these independent estimates may be used to isolate the dependence solely on the mechanical properties of the tissue by reducing or eliminating their dependence on the position of the body part.

Second embodiment

Tissue stiffness may be measured by adding one or more further optical signals using LEDs that emit light of a wavelength that is sensitive to hydration. The processor is adapted to combine the results from all of the optical signals to normalise the data from the one or more additional optical signals so as to provide an estimate of hydration and/or tissue stiffness.

Third embodiment

It is well-known that the wave velocity of the pressure pulse in the artery depends on the stiffness of the artery and the blood pressures, as described by the Moens-Koenig equation. The stiffness of the artery depends on the properties of the artery wall and the stiffness of the tissue surrounding the artery. Blood pressures are found by the means disclosed by the Leman applications and the present application. Arterial stiffness may be found from the data captured by the SAD and PHM (PCT4 Claim 41). The difference between the measured pulse wave velocity and the pulse wave velocity predicted from the blood pressures and arterial stiffness is a measure of how much the arterial stiffness has been modified by the stiffness of tissue surrounding the artery.

Fourth embodiment

The deformation of the tissue under pressure may be measured directly from the average propagation of light in the optical sensor. This uses the DC component of the signal, unlike detection of the luminal area of the artery which uses the AC component. As the body part is pressed harder against the SAD or the SAD pressed harder against the body part, the tissue deforms and changes the cross-sectional area available to transmit the light.

Figure 13 shows the measured ratio of red to green light transmitted as a function of the applied pressure, where the body part is a fingertip. The slope and offset of the line of data points is a measure of the amount of deformation per unit of pressure and thus of stiffness.

The present invention, its aspects and embodiments have been described above purely by way of example only. It will be appreciated by the skilled person that variations of form and function can be made without departing from the scope of the present invention as defined in the following claims.

**Claims**

1. A Personal Health Monitor, comprising:

    a device for acquiring signals which can be used to derive a measurement of the user's blood pressure, the device comprising:

        a blood flow occlusion means (201, 204) adapted to be pressed against one side of a fingertip or to have one side only of a fingertip pressed against it;
        a means for measuring pressure (202) applied by or to a fingertip; and
        an optical sensor (203) for detecting the flow of blood through the fingertip;

    **characterised in that** the Personal Health Monitor further comprises:
    a processor adapted to determine a user's blood pressure throughout the pulse cycle by:

        representing the user's blood pressure through the pulse cycle by a parametric equation;
        predicting luminal area of an artery of the fingertip using a parametric model and the measured pressure;
        finding a set of parameters that minimises the difference between the predicted luminal area of the artery and an area derived from the optical sensor, wherein the set of parameters comprises systolic pressure (401), diastolic pressure (402), rate of rise before systole, rate of fall after systole, time of a dicrotic notice, and amplitude of the dicrotic notch.

2. The Personal Health Monitor of Claim 1, wherein the processor is adapted to use data from several pulse cycles.

3. The Personal Health Monitor of Claim 1, wherein the processor is further adapted to estimate stiffness of tissue in the fingertip from an estimate of a relative position of the artery with respect to the means for measuring pressure, the optical sensor, or both.

4. The Personal Health Monitor of Claim 1, wherein the processor is further adapted to estimate hydration, stiffness, or both for tissue in the fingertip by combining measurements of pulse wave velocity, blood pressure, and arterial stiffness.

5. The Personal Health Monitor of Claim 1, wherein the device is located on the back of a cell phone (301).

6. The Personal Health Monitor of Claim 1, wherein the blood flow occlusion device is adapted to support and locate the sides of the fingertip so as to create a more constant pressure field within the fingertip.

7. The Personal Health Monitor of Claim 1, wherein the means for measuring pressure comprises a pressure sensor immersed in an essentially incompressible gel.

**Patentansprüche**

1. Persönlicher Gesundheitsmonitor, umfassend:
   eine Vorrichtung zum Erfassen von Signalen, die verwendet werden können, um eine Messung des Blutdrucks des Benutzers abzuleiten, wobei die Vorrichtung Folgendes umfasst:

   ein Blutflussokklusionsmittel (201, 204), das dazu ausgelegt ist, gegen eine Seite einer Fingerspitze gedrückt zu werden, oder wobei nur eine Seite einer Fingerspitze dagegen gedrückt werden muss;
   ein Mittel zum Messen des Drucks (202), der von oder auf eine Fingerspitze ausgeübt wird; und
   einen optischen Sensor (203) zum Detektieren des Blutflusses durch die Fingerspitze;
   **dadurch gekennzeichnet, dass** der persönliche Gesundheitsmonitor ferner Folgendes umfasst:
   einen Prozessor, der dazu ausgelegt ist, den Blutdruck eines Benutzers über den gesamten Pulszyklus zu bestimmen durch:

   Repräsentieren des Blutdrucks des Benutzers durch den Pulszyklus durch eine parametrische Gleichung;
   Vorhersagen der Lumenfläche einer Arterie der Fingerspitze unter Verwendung eines parametrischen Modells und des gemessenen Drucks;
   Finden eines Satzes von Parametern, der die Differenz zwischen der vorhergesagten Lumenfläche der Arterie und einer von dem optischen Sensor abgeleiteten Fläche minimiert, wobei der Satz von Parametern systolischen Druck (401), diastolischen Druck (402), Anstiegsrate vor Systole, Abfallrate nach Systole, Zeit einer dikrotischen Kerbe und Amplitude der dikrotischen Kerbe umfasst.

2. Persönlicher Gesundheitsmonitor nach Anspruch 1, wobei der Prozessor dazu ausgelegt ist, Daten von mehreren Pulszyklen zu verwenden.

3. Persönlicher Gesundheitsmonitor nach Anspruch 1, wobei der Prozessor ferner dazu ausgelegt ist, die Steifigkeit von Gewebe in der Fingerspitze aus einer Schätzung einer relativen Position der Arterie in Bezug auf das Mittel zum Messen des Drucks, den optischen Sensor oder beides zu schätzen.

4. Persönlicher Gesundheitsmonitor nach Anspruch 1, wobei der Prozessor ferner dazu ausgelegt ist, Hydratation, Steifigkeit oder beides für Gewebe in der Fingerspitze durch Kombinieren von Messungen der Pulswellengeschwindigkeit, des Blutdrucks und der arteriellen Steifigkeit zu schätzen.

5. Persönlicher Gesundheitsmonitor nach Anspruch 1, wobei sich die Vorrichtung auf der Rückseite eines Mobiltelefons befindet (301).

6. Persönlicher Gesundheitsmonitor nach Anspruch 1, wobei die Blutflussokklusionsvorrichtung dazu ausgelegt ist, die Seiten der Fingerspitze zu stützen und zu lokalisieren, um ein konstanteres Druckfeld innerhalb der Fingerspitze zu erzeugen.

**7.** Persönlicher Gesundheitsmonitor nach Anspruch 1, wobei das Mittel zum Messen des Drucks einen Drucksensor umfasst, der von einem im Wesentlichen inkompressiblen Gel umgeben ist.

**Revendications**

**1.** Moniteur de santé personnel, comprenant :
un dispositif d'acquisition de signaux qui peut être utilisé pour dériver une mesure de la pression artérielle de l'utilisateur, le dispositif comprenant :

un moyen d'occlusion de flux sanguin (201, 204) conçu pour être pressé contre un côté d'un bout de doigt ou pour n'avoir qu'un côté du bout de doigt pressé contre lui ;
un moyen de mesure de la pression (202) appliquée par ou sur un bout de doigt ; et
un capteur optique (203) pour détecter le flux sanguin à travers le bout de doigt ;
**caractérisé en ce que** le moniteur de santé personnel comprend en outre :
un processeur conçu pour déterminer la pression artérielle d'un utilisateur tout au long du cycle du pouls en :

représentant la pression artérielle de l'utilisateur tout au long du cycle du pouls par une équation paramétrique ;
prédisant une surface luminale d'une artère du bout de doigt à l'aide d'un modèle paramétrique et de la pression mesurée ;
trouvant un ensemble de paramètres qui réduit au minimum la différence entre la surface luminale prédite de l'artère et une surface dérivée du capteur optique,
l'ensemble de paramètres comprenant la pression systolique (401), la pression diastolique (402), la vitesse de montée avant systole, la vitesse de descente après systole, le temps de l'incisure dicrote et l'amplitude de l'incisure dicrote.

**2.** Moniteur de santé personnel selon la revendication 1, dans lequel le processeur est conçu pour utiliser des données provenant de plusieurs cycles du pouls.

**3.** Moniteur de santé personnel selon la revendication 1, dans lequel le processeur est en outre conçu pour estimer la rigidité d'un tissu dans le bout de doigt à partir d'une estimation d'une position relative de l'artère par rapport au moyen de mesure de la pression, au capteur optique ou aux deux.

**4.** Moniteur de santé personnel selon la revendication 1, dans lequel le processeur est en outre conçu pour estimer l'hydratation, la rigidité ou les deux pour un tissu dans le bout de doigt en combinant des mesures de vitesse d'onde de pouls, de pression sanguine et de rigidité artérielle.

**5.** Moniteur de santé personnel selon la revendication 1, dans lequel le dispositif est situé à l'arrière d'un téléphone cellulaire (301).

**6.** Moniteur de santé personnel selon la revendication 1, dans lequel le dispositif d'occlusion de flux sanguin est conçu pour supporter et positionner les côtés du bout de doigt de manière à créer un champ de pression plus constant à l'intérieur du bout de doigt.

**7.** Moniteur de santé personnel selon la revendication 1, dans lequel les moyens pour mesurer la pression comprennent un capteur de pression immergé dans un gel essentiellement incompressible.

Figure 1

Figure 2

Figure 3a

Figure 3b

402

401

**Figure 4**

502

501

**Figure 5**

602

603

604      605      601

**Figure 6**

**Figure 7a**

**Figure 7b**

**Figure 7c**

**Figure 7d**

Figure 8

Figure 9

Figure 10

Figure 11

Figure 12

Figure 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013001265 A
- WO 2014125431 A
- WO 2014125355 A
- WO 2016096919 A
- WO 2017140748 A
- WO 2017198981 A

**Non-patent literature cited in the description**

- **LANGEWOUTERS**. *Clin Phys Physiol Meas*, 1986, vol. 7 (1), 43-55
- **DRZEWIECKI**. *Annals of Biomed Eng*, 1994, vol. 22, 88-96
- **BANK**. *Circ Res.*, November 1995, vol. 77 (5), 1008-16
- **STERGIOPULOS**. Physical basis of pressure transfer from periphery to aorta. *Am J Physiol Heart Circ Physiol*, 1998, vol. 274, H1386-H1392